# NOUVEAU FASCICULE DE BREVET EUROPEEN

(11) **EP 1 089 972 B2**
(45) Date de publication et mention de la décision concernant l'opposition: **13.08.2014**
(45) Mention de la délivrance du brevet: 18.08.2004
(21) Numéro de dépôt: 99926540.8
(22) Date de dépôt: 24.06.1999
(51) Int. Cl.: C07D 201/08

(54) **PROCEDE DE VAPORISATION D'AMINONITRILE**
VERFAHREN ZUM VERDAMPFEN VON AMINONITRIL
METHOD FOR EVAPORATING AMINONITRILE

(30) Priorité: 25.06.1998 FR 9808258
(43) Date de publication de la demande: 11.04.2001
(73) Titulaire: Rhodia Opérations, 93306 Aubervilliers (FR)
(72) Inventeur: BOCQUENET, Gérald, 69360 Communay (FR); CHIARELLI, Henri, 69360 Communay (FR); LECONTE, Philippe, 69330 Meyzieu (FR)
(74) Mandataire: Vande Gucht, Anne
(86) Numéro de dépôt international: PCT/FR1999/001524
(87) Numéro de publication internationale: WO 1999/067214

(56) Documents cités:
- EP-A- 0 151 440
- EP-A- 0 659 741
- WO-A-96/22974
- WO-A-98/37063
- DE-A- 1 944 910
- FR-A- 2 755 132
- US-A- 4 628 085

## Description

La présente invention concerne la vaporisation d'aminonitrile et d'eau dans des conditions limitant ou éliminant la formation de sous-produits lourds notamment d'oligomères d'acide amino-carboxytique.

La réaction entre un aminonitrile et l'eau conduit à la formation de lactame, en particulier du caprolactame dans le cas de la mise en oeuvre d'amino-6 capronitrile.

Cette réaction peut être réalisée en phase liquide à chaud et sous une pression élevée. Elle peut également être effectuée en phase vapeur. Pour ce deuxième mode de réalisation, il est donc nécessaire de transformer l'aminonitrile et l'eau à l'état de vapeur. A titre d'exemples de réalisation d'hydrolyse d'aminonitrile en phase vapeur, on peut se référer notamment au brevet EP-A-0 659 741 et à la demande internationale WO-A-96/22974.

Le choix du mode de vaporisation de l'aminonitrile et de l'eau n'est pas trivial.

En effet, il peut être envisagé de réaliser un mélange liquide eau/aminonitrile, puis de chauffer ce mélange à une température suffisante pour vaporiser les deux constituants. On observe alors la formation de composés lourds à fonction amide ou acide carboxylique salifiée (oligomères). Ces composés sont susceptibles de se fixer au moins en partie sur le catalyseur et ainsi de diminiuer sa durée de vie. D'autre part, ils se déposent dans l'appareillage et l'encrassent. Cela nécessite le nettoyage périodique dudit appareillage, donc l'arrêt de l'installation de manière relativement fréquente, avec toutes les conséquences économiques que l'on imagine.

Une autre technique envisageable serait de vaporiser séparément les flux d'aminonitrile et d'eau. Il a été constaté par la Demanderesse qu'aux températures nécessaires pour vaporiser l'aminonitrile, celui-ci se dégrade en proportions non négligeables, pour donner un composé de type amidine ou polyamidine (condensation de plusieurs molécules d'aminonitrile avec élimination d'ammoniac).

Pour éviter ces différents inconvénients, il a maintenant été trouvé un procédé de vaporisation d'aminonitrile et d'eau, caractérisé en ce que l'eau à l'état de vapeur sert de gaz vecteur pour cette vaporisation.

La dégradation de l'aminonitrile dépend de la température d'évaporation et du temps de séjour du liquide pendant son évaporation. Ainsi dans le procédé de l'invention, le temps de séjour du liquide est minimisé par la technologie de l'évaporateur, et l'eau à l'état de vapeur diminue la pression partielle de l'aminonitrile, ce qui abaisse sa température d'évaporation.

Le rapport molaire eau/aminonitrile peut varier très largement dans le procédé de l'invention. Il dépend essentiellement du procédé d'hydrolyse cyclisante dans lequel les réactifs seront engagés. Ce rapport molaire entre l'eau et l'aminonitrile engagés se situe habituellement entre 0,5 et 100 et de préférence entre 1 et 20. La valeur supérieure de ce rapport n'est pas critique pour l'invention, mais des rapports plus élevés n'ont guère d'intérêt pour la réaction d'hydrolyse, pour des questions économiques.

De manière générale, la vapeur d'eau sera à une température de 120°C à 600°C et de préférence de 200°C à 550°C.

L'aminonitrile sera généralement mis en oeuvre à une température de 20°C à 300°C. De préférence, cette température sera de 100°C à 250°C.

Le mélange aminonitrile/vapeur d'eau est porté rapidement dans un échangeur thermique, à une température où la vaporisation du mélange est totale.

Cette température peut être le cas échéant celle à laquelle sera réalisée la réaction entre l'aminonitrile et l'eau. Une telle température de réaction se situe habituellement entre 200°C et 450°C et de préférence entre 250°C et 400°C.

La pression absolue a laquelle est réalisée la vaporisation de l'aminonitrile est généralement de 0.1 à 3 bar.

L'aminonitrile utilisé dans le procédé de l'invention est un aminonitrile aliphatique, linéaire ou ramifié, ayant de 3 à12 atomes de carbone.

A titre d'exemples, on peut citer plus particulièrement les aminonitriles aliphatiques provenant de l'hydrogénation en fonction amine primaire d'une des deux fonctions nitrites des dinitriles tels que l'adiponitrile, le methylglutaronitrile, l'éthylsuccinonitrile, le dimethylsuccinonitrile, le malononitrile, le succinonitrile, le glutaronitrile, le dodecanedinitrile.

L'aminonitrile le plus important est l'amino-6 capronitrile dont l'hydrolyse cyclisante conduit au caprolactame, dont la polymérisation fournit le polyamide 6.

Par commodité, dans ce qui suit, on pourra se référer plus particulièrement à l'amino-6 capronitrile (ou ACN).

La mise en oeuvre du procédé est réalisée à l'aide d'un système sans rétention de liquide.

Les technologies qui sont retenues pour limiter le temps de séjour du produit en phase liquide pendant l'évaporation sont de deux types:
- évaporation en film de l'aminonitrile sur une surface chauffée,
- évaporation d'un brouillard d'aminonitrile au moins partiellement liquide dans la vapeur d'eau surchauffée, dans ce cas les contacts liquide/paroi chaude sont remplacés par un contact gaz/gouttes de liquide.

Dans le cas d'une évaporation en film sur une surface chauffée, la chaleur nécessaire à l'évaporation est amenée d'une part par la chaleur sensible de la vapeur et de aminonitrile et d'autre part par transfert de chaleur au travers de la surface d'évaporation. L'évaporateur est du type évaporateur à film tombant.

La distribution du liquide sur les tubes de l'évaporateur peut se faire selon les systèmes de distribution généralement utilisés dans ce type de technologie:
- alimentation de l'aminonitrile au moins partiellement liquide sur la plaque tubulaire, puis distribution de cet aminonitrile dans chaque tube,
- distribution de l'aminonitrile au moins partiellement liquide dans chaque tube par pulvérisation en brouillard de ce dernier au dessus de la plaque tubulaire;
cette technologie présente par rapport à la précédente l'avantage de diminuer encore le temps de séjour en phase liquide à haute température; la pulvérisation du liquide peut se faire par l'intermédiaire d'une buse alimentée par le liquide uniquement ou mieux par une buse alimentée simultanément par le liquide et la vapeur d'eau.

Dans le cas d'une évaporation en brouillard par contact gaz/gouttes de liquide, la chaleur est totalement apportée par la chaleur sensible des deux constituants, l'aminonitrile au moins partiellement en phase liquide et l'eau en phase vapeur.

La température de la vapeur d'eau et celle de l'aminonitrile au moins partiellement liquide sont choisies de telle façon que le brouillard obtenu soit à une température égale ou supérieure au point de rosée du mélange eau/aminonitrile constituant ledit brouillard. Le point de rosée dépend bien évidemment du rapport eau/aminonitrile et est facilement déterminé pour le rapport choisi.

Ainsi, à titre d'exemple, à pression atmosphérique, le point de rosée est de 180°C pour un rapport molaire eau/amino-6 capronitrile (ACN) de 4, de 110°C pour un rapport molaire eau/ACN de 56, de 210°C pour un rapport eau/ACN de 1 et de 230°C pour l'ACN pur.

Cette évaporation en brouillard par contact gaz/gouttes de liquide peut être monoétagée ou multiétagée. Dans le cas où l'évaporation est monoetagée,la température de l'aminonitrile et de la vapeur d'eau sont telles que la vaporisation du liquide peut être totale ou partielle. Si l'évaporation est multiétagée, le flux d'aminonitrile, préchauffé à 230°C, par exemple, est subdivisé en plusieurs parties, trois ou quatre;
la première partie de ce liquide est mélangée a la vapeur d'eau surchauffée, à 300°C par exemple, de telle manière que la totalité du liquide se vaporise, la température du mélange baissant simultanément aux environs du point de rosée du fait de la vaporisation. Le mélange à l'état vapeur est ensuite surchauffé, a 300°C par exemple, puis remélangé à la seconde partie du liquide qui se vaporise à son tour; le processus est répété autant de fois que nécessaire pour obtenir la vaporisation totale du liquide. Dans ce procédé, le brouillard de liquide est généré à chaque étage par des buses de pulvérisation, le mélange étant ensuite realisé dans un volume suffisant pour assurer la vaporisation totale du liquide.

Le système de vaporisation de l'aminonitrile sera choisi de préférence de telle façon que la durée de présence d'aminonitrile liquide dans ledit système, comprenant
le préchauffage dudit aminonitrile, soit inférieur ou égal à une minute, préférentiellement inférieur ou égal à 5 secondes.

Les exemples qui suivent illustrent la présente invention.

### EXEMPLE 1

200 g/h d'amino-6 capronitrile (ACN) préchauffé à 230°C et 129 g/h de vapeur d'eau à 300°C sont injectés au travers d'une buse de 1 mm.

Le brouillard ainsi formé est vaporisé, puis surchauffé à 300°C à l'aide d'un échangeur, avant d'alimenter un réacteur d'hydrolyse contenant 162 g d'alumine, ledit réacteur étant maintenu à 300°C.

Sur plus de 400 h de fonctionnement, aucun encrassement du réacteur, ni aucune diminution de l'activité catalytique (mesurée par le taux de transformation de l'ACN à débit constant égal à 99 %) n'ont été constatés.

### ESSAI COMPARATIF 1

329 g/h d'un mélange ACN/eau à 61 % en poids d'ACN sont alimentés dans un évaporateur de 200 ml chauffé à 300°C.

Le mélange gazeux sortant de l'évaporateur est envoyé dans un réacteur d'hydrolyse contenant 162 g d'alumine, ledit reacteur étant maintenu à 300°C.

L'essai est arrêté après 172 h de fonctionnement. Pendant cette période, le taux de transformation de l'ACN est passé de 99 % à 95 %.

Après démontage de l'appareillage, on constate la présence d'un solide (polyamide 6) à l'intérieur de l'évaporateur, ainsi qu'à l'entrée du réacteur d'hydrolyse (25% de la hauteur du contenu dudit réacteur sont pris en masse).

## Revendications

1. Procédé de vaporisation d'aminonitrile aliphatique, linéaire ou ramifié, ayant de 3 à 12 atomes de carbone et d'eau, **caractérisé en ce que**:
- l'eau à l'état de vapeur sert de gaz vecteur pour la vaporisation.
- il est mis en oeuvre avec un système sans rétention de liquide.
- on utilise l'une des technologies suivantes:
- évaporation en film de l'aminonitrile sur une surface chauffée, dans un évaporateur de type film tombant;
- évaporation d'un brouillard d'aminonitrile au moins partiellement liquide dans la vapeur d'eau surchauffée.

2. Procédé selon la revendication 1 , **caractérisé en ce que** la vapeur d'eau est mise en oeuvre à une température de 200°C à 550°C.

3. Procédé selon l'une des revendications 1 ou 2, **caractérisé en ce que** l'aminonitrile est mis en oeuvre à une température de 100°C à 250°C.

4. Procédé selon l'une des revendications 1 à 3 **caractérisé en ce que** le mélange d'aminonitrile dans la vapeur d'eau obtenu est porté à la température de réaction entre l'aminonitrile et l'eau.

5. Procédé selon la revendication 4, **caractérisé en ce que** la température de réaction est comprise entre 200°C et 450°C, de préférence entre 250°C et 400°C.

6. Procédé selon l'une des revendications 1 à 5, **caractérisé en ce que** l'aminonitrile provient de l'hydrogénation en fonction amine primaire de l'une des deux fonctions nitriles d'un dinitrite choisi parmi l'adiponitrile, le méthylglutaronitrile, l'éthylsuccinonitrile, le diméthylsuccinonitrile, le malononitrile, le succinonitrile, le glutaronitrile, le dodécanedinitrile et est de préférence l'amino-6 capronitrile.

7. Procédé selon l'une des revendications 1 à 6, **caractérisé en ce que** la vaporisation de l'aminonitrile est réalisée sous une pression absolue de 0,1 à 3 bar.

8. Procédé selon l'une des revendications 1 à 7, **caractérisé en ce que** l'évaporation d'un brouillard d'aminonitrile au moins partiellement liquide dans la vapeur d'eau surchauffée est monoétagée ou multiétagée.

9. Procédé selon l'une des revendications 1 à 8, **caractérisé en ce que** le système de vaporisation de l'aminonitrile est choisi de telle façon que la durée de présence d'aminonitrile liquide dans ledit système soit inférieur ou égal à une minute, préférentiellement inférieur ou égal à 5 secondes.

## Patentansprüche

1. Verfahren zum Verdampfen von linearem oder verzweigtem aliphatischem Aminonitril mit 3 bis 12 Kohlenstoffatomen, **dadurch gekennzeichnet, daß**
- das Wasser im Zustand von Dampf als Trägergas für die Verdampfung dient,
- es mit einem System ohne Zurückhalten von Flüssigkeit durchgeführt wird,
- man eine der folgenden Techniken anwendet:
- Filmverdampfen des Aminonitrils auf einer erhitzten Fläche in einem Fallfilmverdampfer;
- Verdampfen eines Nebels von Aminonitril, das mindestens teilweise flüssig ist, in überhitztem Wasserdampf.

2. Verfahren nach Anspruch 1, **dadurch gekennzeichnet, daß** der Wasserdampf bei einer Temperatur von 200°C bis 550°C eingesetzt wird.

3. Verfahren nach einem der Ansprüche 1 oder 2, **dadurch gekennzeichnet, daß** das Aminonitril bei einer Temperatur von von 100°C bis 250°C eingesetzt wird.

4. Verfahren nach einem der Ansprüche 1 bis 3, **dadurch gekennzeichnet, daß** die erhaltene Mischung von Aminonitril in Wasserdampf auf die Temperatur der Reaktion zwischen Aminonitril und Wasser gebracht wird.

5. Verfahren nach Anspruch 4, **dadurch gekennzeichnet, daß** die Temperatur der Reaktion zwischen 200°C und 450°C, vorzugsweise zwischen 250°C und 400°C, beträgt.

6. Verfahren nach einem der Ansprüche 1 bis 5, **dadurch gekennzeichnet, daß** das Aminonitril aus der Hydrierung einer der beiden Nitrilfunktionen eines Dinitrils, das unter Adiponitril, Methylglutaronitril, Ethylsuccinonitril, Dimethylsuccinonitril, Malononitril, Succinonitril, Glutaronitril, Dodecandinitril ausgewählt ist und vorzugsweise 6-Aminocapronitril ist, zur primären Aminfunktion stammt.

7. Verfahren nach einem der Ansprüche 1 bis 6, **dadurch gekennzeichnet, daß** die Verdampfung des Aminonitrils unter einem absolutem Druck von 0,1 bis 3 bar realisiert wird.

8. Verfahren nach einem der Ansprüche 1 bis 7, **dadurch gekennzeichnet, daß** das Verdampfen eines Nebels von mindestens teilweise flüssigem Aminonitril in dem überhitzten Wasserdampf in nur einer Stufe oder in mehreren Stufen erfolgt.

9. Verfahren nach einem der Ansprüche 1 bis 8, **dadurch gekennzeichnet, daß** das System zum Verdampfen des Aminonitrils in der Weise ausgewählt wird, daß die Dauer der Anwesenheit von flüssigem Aminonitril in dem System kleiner gleich eine Minute, vorzugsweise kleiner gleich 5 Sekunden, ist.

## Claims

1. Process for vaporizing a linear or branched aliphatic aminonitrile having 3 to 12 carbon atoms and water, **characterized in that**:
- water in the vapour state is used as the carrier gas for the vaporization,
- it is performed with a system without retention of liquid,
- one of the following technologies is employed:
- evaporation of the aminonitrile as a film on a heated surface, in an evaporator of falling-film type;
- evaporation of an at least partly liquid mist of aminonitrile in the superheated water vapour.

2. Process according to Claim 1, **characterized in that** the water vapour is employed at a temperature of from 200 to 550°C.

3. Process according to either of Claims 1 and 2, **characterized in that** the aminonitrile is employed at a temperature of from 100 to 250°C.

4. Process according to one of Claims 1 to 3, **characterized in that** the mixture of aminonitrile in the water vapour that is obtained is brought to the temperature of reaction between the aminonitrile and the water.

5. Process according to Claim 4, **characterized in that** the reaction temperature is between 200 and 450°C, preferably between 250 and 400°C.

6. Process according to one of Claims 1 to 5, **characterized in that** the aminonitrile originates from the hydrogenation to a primary amine function of one of the two nitrile functions of a dinitrile selected from adiponitrile, methylglutaronitrile, ethylsuccinonitrile, dimethylsuccinonitrile, malononitrile, succinonitrile, glutaronitrile and dodecanedinitrile and is preferably 6-amino-capronitrile.

7. Process according to one of Claims 1 to 6, **characterized in that** the vaporization of the aminonitrile is conducted under an absolute pressure of from 0.1 to 3 bar.

8. Process according to one of Claims 1 to 7, **characterized in that** the evaporation of an at least partly liquid mist of aminonitrile in the superheated water vapour is single-stage or multi-stage.

9. Process according to one of Claims 1 to 8, **characterized in that** the system for vaporizing the aminonitrile is selected such that the dwell time of liquid aminonitrile in the said system is less than or equal to one minute, preferably less than or equal to 5 seconds.
